# NEW EUROPEAN PATENT SPECIFICATION

(11) **EP 0 905 257 B2**
(45) Date of publication and mention of the opposition decision: **21.02.2007**
(45) Mention of the grant of the patent: 19.05.2004
(21) Application number: 98202972.0
(22) Date of filing: 04.09.1998
(51) Int. Cl.: C12P 41/00, C12P 13/04, C07C 231/06, C07C 253/00, C07C 255/24, C07C 237/04, C07C 229/22, C07D 317/28, C07D 317/30, C07D 319/06

(54) **Process for preparing optically active 2-amino-Omega-oxoalkanoic acid derivatives**
Verfahren zur Herstellung von optisch-aktiven 2-omega-oxoalkansäurederivaten
Procédé de préparation de dérivés optiquement actifs d'acides 2-amino-omega-oxoalcanoiques

(30) Priority: 25.09.1997 NL 1007113
(43) Date of publication of application: 31.03.1999
(73) Proprietor: DSM IP Assets B.V., 6411 TE Heerlen (NL)
(72) Inventor: Boesten, Wilhelmus Hubertus Joseph, 6132 BJ Sittard (NL); Broxterman, Quirinus Bernardus, 6151 GE Sittard (NL); Plaum, Marcus Joseph Maria, 6141 AC Sittard (NL)

(56) References cited:
- EP-A- 0 052 200
- EP-A- 0 052 201
- EP-A- 0 307 023
- EP-A- 0 383 403
- EP-A- 0 919 630
- US-A- 5 332 826
- SHAM, HING L. ET AL: "Novel non-basic bioisostere of histidine synthesized from L-aspartic acid" J. CHEM. SOC., CHEM. COMMUN. (1987), (23), 1792-3 CODEN: JCCCAT;ISSN: 0022-4936,1987, XP002065169
- DATABASE WPI Section Ch, Week 7422 Derwent Publications Ltd., London, GB; Class E17, AN 74-40592V XP002065170 & JP 48 039416 A (MITSUBISHI CHEM IND LTD)

## Description

The invention relates to a process for preparing an optically active 2-amino-ω-oxoalkanoic acid derivative of formula 1, 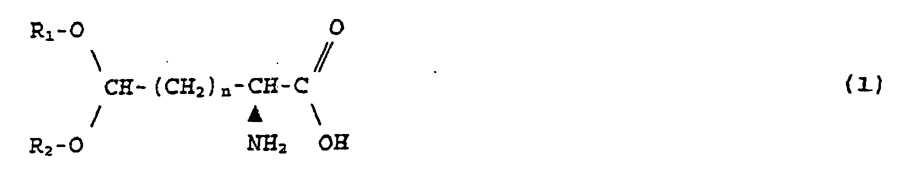 in which n equals 0, 1, 2, 3 or 4 and R₁ and R₂ each independently represent an alkyl group with 1-10 C atoms or form a ring with 3 or 4 C atoms together with the O atoms to which they are bound and the C atom to which the O atoms are bound.

The preparation of a racemic mixture of a 2-amino-ω-oxoalkanoic acid derivative of formula 1 is described in Biorg. & Med. Chem. (1995), 1237-1240. The preparation method described therein however proceeds via an 8-step process, starting from 3,4-dihydro-2H-pyran, with various protection and de-protection steps and is hence very laborious.

The invention provides a new, simple concept for preparing 2-amino-ω-oxoalkanoic acid derivatives.

This is achieved according to the invention with a process in which the corresponding aldehyde of formula 2 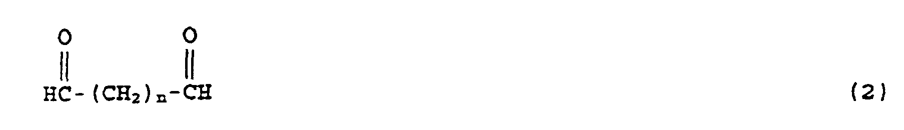 with n as described above is converted into the corresponding acetal-protected aldehyde of formula 3 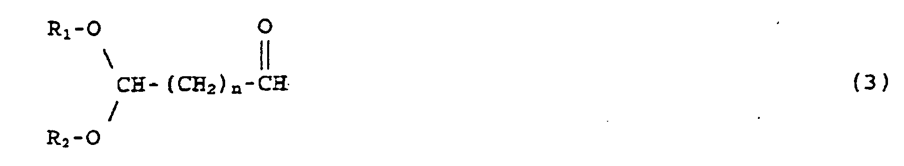 with n, R₁ and R₂ as described above, the acetal-protected aldehyde is converted into the corresponding aminonitrile of formula 4 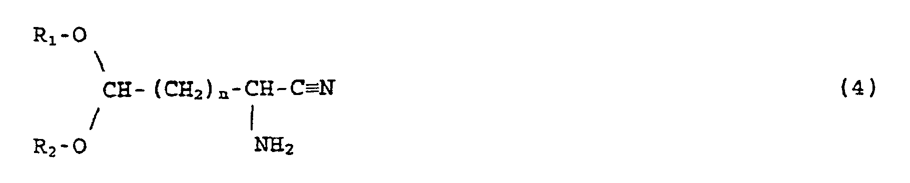 with n, R₁ and R₂ as described above, the aminonitrile is converted into the corresponding amino acid amide of formula 5 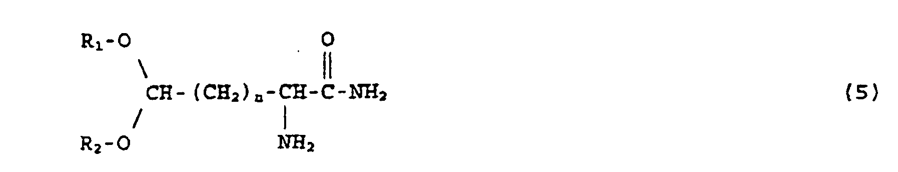 with n, R₁ and R₂ as described above, the amino acid amide is subjected to an enzymatic, enantioselective hydrolysis in which the R enantiomer of the amino acid amide remains and the S enantiomer is converted into the S amino acid, and the S amino acid is isolated.

It has been found that, in spite of the fact that the selectivity in the 1st step of the process is relatively low, an economically attractive process can nevertheless be obtained.

The optically active compounds of formula 1 are new and are particularly suitable for use in the preparation of, for example, allysin, an important crosslinker in proteins, as described in Int. J. Pept. Protein Res. (1988), 307-20, and in the preparation of pharmaceuticals, for example as described in EP-A-629627.

EP-A-0383403 and EP-A-0307023 both relate to the stereoselective preparation of amino acids. The amino acids are prepared from the respective amino acid amides, which are converted by an amidase to amino acids. The other enantiomer can either be converted to its amino acid form via a Schiff base with benzaldehyde or converted to its other enantiomer by a racemase.

EP-A-0052201 dicloses the preparation of a racemic mixture of 1,3-diox(ol)an-2yl-propionic acid from 1,3-diox (ol)an-2yl-propenal, using HCN, NH₄ and CO₂ followed by hydrolysis with OH⁻.

JP 48 039416 A discloses the preparation of 1,3-dioxolan-2yl butanal from glutaraldehyde and glycol.

GB-A-1124149 discloses the preparation of amino acids starting from aldehydes by the reaction of HCN and NH₄ followed by hydrolysis of the so obtained α-amino-nitrile. JP-A-57158743 discloses the preparation of α-amino acid-amide by contacting α-amino-nitrile with NaOH and acetone.

In the process according to the invention one of the two aldehyde functions in the aldehyde of formula 2 is first protected through conversion, in a manner known per se into an acetal. This can for example be done with the aid of an alcohol, for example an alcohol with 1-5 C atoms when R₁ and R₂ represent an alkyl group, or with the aid of a diol, in particular a 1,2-ethanediol or a 1,3-propanediol, whether or not substituted with for example an alkyl group with 1-5 C atoms, for example 1,2-ethanediol, 1,2-propanediol, 1,3-propanediol or 2,3-butanediol, when R₁ and R₂ form part of a ring structure; or via re-acetalization, for example with the aid of ortho-formate esters.

The acetalization can for example be carried out by bringing the aldehyde of formula 2 into contact with an alcohol or a diol under acid conditions, for example in the presence of a sulphonic acid, in particular p-toluenesulphonic acid. The acetalization is optionally carried out in the presence of a solvent. In principle, any solvent that does not interfere with the reaction can be used as a solvent, for example aromatic hydrocarbons, in particular benzene, toluene and xylene; halogenated hydrocarbons, for example dichloromethane; esters, preferably hindered esters, in particular isopropyl acetate or isobutyl acetate and ethers, in particular methyl-t-butyl ether (MTBE). The acetalization with an alcohol or diol is preferably carried out at elevated temperature, for example at a temperature of between 50 and 150°C, preferably at reflux temperature.

The acetal-protected aldehyde obtained is subsequently converted into the corresponding aminonitrile, for example via the Strecker chemistry known per se. To this end the acetal-protected aldehyde can for example be converted into the aminonitrile in the presence of ammonia with the aid of a cyanide compound, for example HCN, NaCN or KCN.

The aminonitrile is subsequently converted into the corresponding racemic amino acid amine, for example, as described in GB-A-1548032, by converting the aminonitrile at a pH of between 11 and 14, preferably between 12.5 and 13.5, in the presence of a base and a ketone or aldehyde, optionally followed by hydrolysis of the intermediately formed Schiff base in the presence of water. Preferably, KOH or NaOH or a corresponding base is used as the base and an aliphatic ketone, for example acetone, methyl ethyl ketone or cyclohexanone, or an aromatic aldehyde, for example benzaldehyde, as the ketone or aldehyde.

After the conversion of the aminonitrile into the amino acid amide, the reaction mixture is preferably first subjected to a treatment with an aldehyde, for example a benzaldehyde, upon which the Schiff base of the amino acid amide is obtained and, any racemic amino acid formed remains in solution. Such a treatment presents the advantage that the desired (S) amino acid can be obtained in a higher enantiomeric excess.

Preferably, a benzaldehyde is used in the formation of the Schiff base of the amino acid amide.

An advantage of benzaldehyde is that it is easy to separate the Schiff base and recover the benzaldehyde. Another advantage of benzaldehyde is that it is not miscible with water, as a result of which it is also far more preferable than other extraction means for use as extraction means because the formed Schiff base of the optically active amino acid amide dissolves in the benzaldehyde and the other components of the reaction mixture in the water phase. It has surprisingly been found that the hydrolysis of the Schiff base of the amino acid amide resulting in the salt of the amino acid amide can be carried out without the acetal function deteriorating significantly.

'Benzaldehyde' is also understood to include substituted benzaldehydes such as lower (1-4 C) alkylbenzaldehydes, halogenbenzaldehydes, nitrobenzaldehydes and lower (1-4 C) alkoxybenzaldehydes.

The reaction with benzaldehyde resulting in the formation of a Schiff base can for example be carried at a temperature of between 20 and 60°C, preferably between 35 and 45°C. If equimolar amounts of benzaldehyde, for example 0.9-2, in particular 0.95-1.1 equivalents relative to the amino acid amide, are used in the formation of the Schiff base without a different solvent for the Schiff base of the amide, a precipitate of the Schiff base of the amino acid amide is obtained. The other components remain dissolved in the mother liquor. If an excess of benzaldehyde is used, the benzaldehyde acts not only as a reaction means, but also as a solvent, and two layers are obtained. It is also possible to use mixtures of benzaldehyde and other solvents, for example mixtures with aromatic hydrocarbons, for example toluene, ketones, for example methyl isobutyl ketone, halogenated hydrocarbons, for example chloroform or dichloromethane; esters, for example ethyl acetate and butyl acetate. The organic phase can subsequently be used as such in the hydrolysis of the Schiff base of the amino acid amide to the amino acid amide or it can be subjected to concentration, upon which the Schiff base of the amino acid amide precipitates as a solid.

The amino acid amide can be recovered from the corresponding Schiff base in a simple manner, through acidification with a strong acid, for example sulphuric acid, until a pH of between 3 and 5, preferably between 3.5 and 4.5 has been obtained, with the Schiff base decomposing to form the aldehyde and the corresponding salt of the amino acid amide.

The free amino acid amide can subsequently be obtained from the salt through treatment with a base, for example with triethylamine. Preferably, the conversion of the salt into the free amino acid amide is carried out with the aid of a (strongly) basic ion exchanger, for example Amberlyst 26^{™} or IRA 900^{™}.

The amino acid amides of formula 5 and the Schiff bases thereof of formula 6 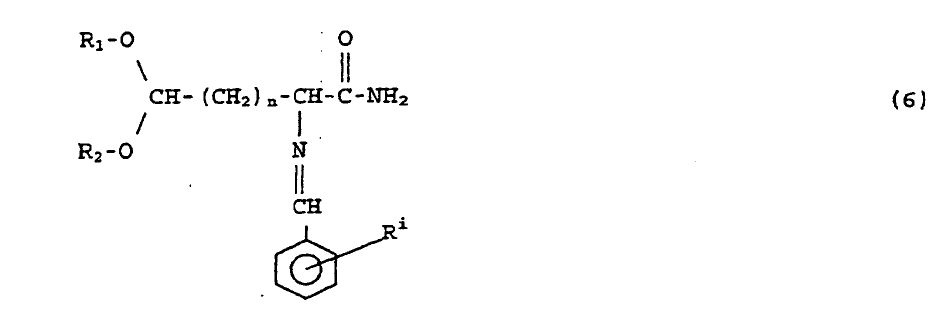 that are obtained as intermediates are new compounds per se. The invention hence also relates to these intermediates, both in racemic form and in optically active form, in particular the amino acid amides having an e.e. greater than 80%, preferably greater than 90%, more preferably greater than 95%, most preferably greater than 98%, in particular greater than 99%.

The amino acid amide is subsequently subjected to an enantioselective, enzymatic hydrolysis in which the S enantiomer is selectively converted into the corresponding acid and the R enantiomer remains unaffected. The (S)-2-amino-ω-oxoalkanoic acid derivative can then be obtained with an e.e. of more than 90%, in particular more than 95%, preferably more than 98%, in particular more than 99%. The enantioselective enzymatic hydrolysis is preferably carried out in an aqueous environment. It is however also possible to use an organic solvent. The temperature is not particularly critical and lies, for example, between 0 and 60°C, preferably between 20 and 50°C. The pH at which the enzymatic hydrolysis is carried out is preferably between 5 and 10.5, in particular between 8.0 and 9.5. An amidase, for example an amidase derived from the genus Aspergillus, Mycobacterium, Aeromonas, Bacillus, Pseudomonas or Ochrobactrum, is suitable for use as the enzyme. Preferably, an amidase derived from Pseudomonas putida or from Ochrobactrum anthropi is used.

After for example the removal of the (R) enantiomer of the amino acid amide as the Schiff base, or concentration and treatment with alcohol, for example isopropanol, the optically active (S)-2-amino-ω-oxoalkanoic acid derivative can be obtained. The Schiff base of the (R) enantiomer of the amino acid amide can optionally be converted into the free amino acid amide, which in turn can optionally be hydrolyzed under mild conditions, for example via a (non-stereoselective) enzymatic hydrolysis as described in EP-A-179523, using Rhodococcus erythropolis or an extract thereof, to obtain the (R)-2-amino-ω-oxoalkanoic acid derivative. The optically active (R)-2-amino-ω-oxoalkanoic acid derivatives are new compoundsi with various very interesting applications. The compounds of formula 7 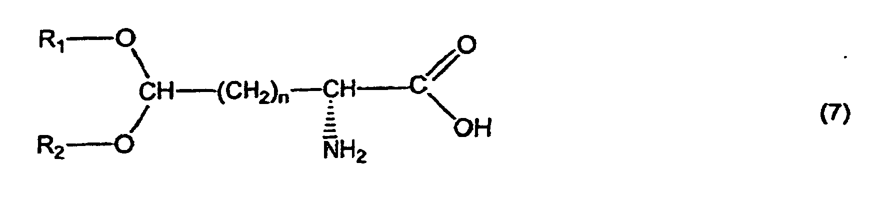 are for example very suitable for use in the preparation of D-pipecolic acid derivatives as for example described in J. O.C. (1990), 5551-3, and in Bioorg. & Med. Chem. (1995), 1237-1240, which are in turn used perse in the preparation of various pharmaceuticals as for example described in EP-A-672665, DE-A-3702943 and US-A-5409946. Another interesting use of the compounds of formula 7 is the use as intermediate in the preparation of D-proline, which is used for example in the preparation of Elitriptan as described in 'Drugs of the Future' (1997), 221-223, herein incorporated by reference. The invention also relates to these new compounds, in particular to the optically active (R)-2-amino-ω-oxoalkanoic acid derivatives having an e.e. greater than 80%, preferably greater than 90%, in particular greater than 95%.

The invention will now be elucidated with reference to the examples without being limited thereby.

### Example I

### Preparation of 4-(1,3-dioxolane-2-yl)-1-butanal via the acetalization of glutardialdehyde with ethylene glycol

Toluene (1 litre), ethylene glycol (155 grammes, 2.5 mol) and p-toluenesulphonic acid (1 gramme) were successively dosed to a 50% solution of glutardialdehyde in water (500 grammes, 2.5 mol). The mixture was heated to reflux temperature. The water was azeotropically removed with the aid of a Dean-Stark apparatus. As soon as all the water had been removed with the aid of the Dean-Stark apparatus (approx. 6 hours) the solution was cooled to room temperature.

Sodium bicarbonate (2.1 grammes) and water (250 ml) were added to the solution. After half an hour's vigorous stirring the layers were separated. This washing of the toluene phase was repeated twice. Then the toluene phase was evaporated in a rotary film evaporator to obtain a pale yellow oil. The 4-(1,3-dioxolane-2-yl)-1-butanal content was 50% (G.C.). Yield = 35%.

### Example II

### Preparation of the Schiff base of 2-amino-5-(1,3-dioxolane-2-yl)-pentanoic acid amide via the Strecker reaction of 4-(1,3-dioxolan-2-yl)-1-butanal

Sodium cyanide (49 grammes, 1 mol) and ammonium acetate (77 grammes, 1 mol) were successively added to a 25% solution of ammonia in water (500 ml). In one hour's time the oil obtained in Example 1 (288 grammes, 1 mol) was added dropwise to this solution. After 5 hours' further stirring, acetone (100 ml) and a 45% solution of potassium hydroxide in water (10 ml) were successively dosed to this solution. After 3 hours glacial acetic acid (7 ml) was added. The solution was concentrated to approx. 400 grammes with the aid of a rotary film evaporator. Water (600 ml) and toluene (150 ml) were added to the concentrate. After half an hour's stirring the layers were separated. Benzaldehyde (74 grammes) was slowly dosed to the water phase with vigorous stirring. The white precipitate formed was removed through filtration and washed with water. After drying, the yield of the Schiff base of 2-amino-5-(1,3-dioxolane-2-yl)-pentanoic acid amide was 195 grammes (yield 70%). Purity >98% (¹H-NMR).

### Example III

### Preparation of (S)-2-amino-5-(1,3-dioxolane-2-yl)-pentanoic acid via the enzymatic resolution on 2-amino-5-(1,3-dioxolane-2-yl)-pentanoic acid amide

The Schiff base of 2-amino-5-(1,3-dioxolane-2-yl)-pentanoic acid amide (240 grammes, 0.87 mol) was suspended in toluene (1000 ml) and water (1500 ml). Concentrated sulphuric acid (44.5 grammes, 0.44 mol) was very slowly dosed with vigorous stirring, so that the pH remained above 4.0. After the dosage of the sulphuric acid the layers were separated. The water phase was subsequently passed over a strongly basic ion exchanger (type IRA 900). The sulphate-free water phase was subsequently brought to a pH of 9.0 with acetic acid. Pseudomonas putida (40 grammes) was added to this solution. After 8 hours' stirring at 37°C decalite (30 grammes) and, dropwise, benzaldehyde (51 grammes, 0.48 mol) were successively dosed to the suspension. The precipitate formed was removed with the aid of filtration. The filtrate obtained was concentrated to about 450 grammes with the aid of a rotary film evaporator. After heating to 65°C isopropanol (900 grammes) was added to the solution. After slow cooling to -5°C the white crystals formed were filtered and successively washed with ice water and isopropanol.
Yield of (S)-2-amino-5-(1,3-dioxolane-2-yl)-pentanoic acid: 42 grammes (26%).
Melting point = 258°C.
Purity > 99% (titration)
E.e. > 99% (HPLC).

### Example IV

### Preparation of (S)-2-amino-5-(1,3-dioxolane-2-yl)-pentanoic acid via the enzymatic resolution on 2-amino-5-(1,3-dioxolane-2-yl)-pentanoic acid amide

Glacial acetic acid was added to the water phase obtained after the toluene extraction (see Example II) so that the pH became 9.0. At 37°C

Pseudomonas putida was added to this water phase. After 8 hours' stirring the water phase was treated as described in Example III.
Yield of (S)-2-amino-5-(1,3-dioxolane-2-yl)-pentanoic acid = 11%.
Purity = 95% (HPLC), e.e. > 99% (HPLC).

### Example V

### Preparation of (S)-2-amino-5-(1,3-dioxolane-2-yl)-pentanoic acid via the enzymatic resolution on 2-amino-5-(1,3-dioxolane-2-yl)-pentanoic acid amide

The water phase obtained after the hydrolysis of the Schiff base with sulphuric acid (see Example III) was brought to a pH of 9.0 with the aid of triethylamine. At 37°C Pseudomonas putida was subsequently dosed to this solution and after 8 hours' stirring it was processed further as described in Example III.
Yield of (S)-2-amino-5-(1,3-dioxolane-2-yl)-pentanoic acid = 24%.
Purity = 76% (HPLC), e.e. > 99% (HPLC).

## Claims

1. Process for preparing an (S)-2-amino-ω-oxoalkanoic acid derivative of formula 1, 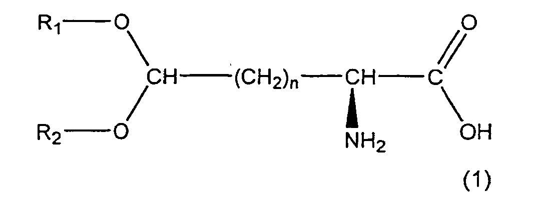 in which n equals 0, 1, 2, 3 or 4 and R₁ and R₂ each independently represent an alkyl group with 1-10 C atoms or form a ring with 3 or 4 C atoms together with the O atoms to which they are bound and the C atom to which the O atoms are bound, **characterized in that** the corresponding aldehyde of formula 2 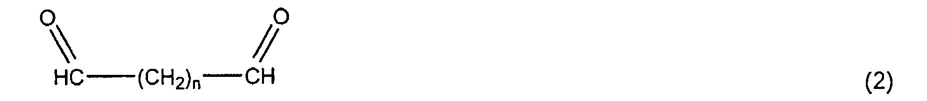 with n as described above is converted into the corresponding acetal-protected aldehyde of formula 3 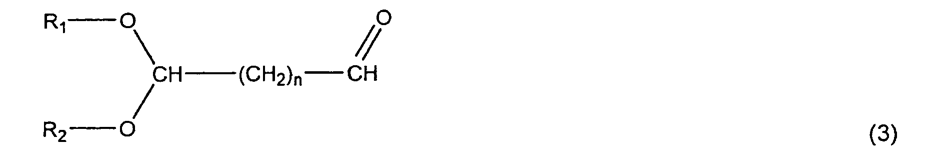 with n, R₁ and R₂ as described above, the acetal-protected aldehyde is converted into the corresponding aminonitrile of formula 4 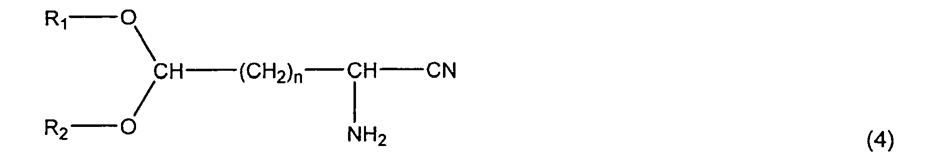 with n, R₁ and R₂ as described above, the aminonitrile is converted into the corresponding amino acid amide of formula 5 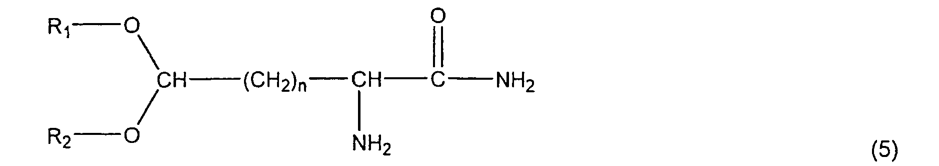 with n, R₁ and R₂ as described above, the amino acid amide is subjected to an enzymatic, enantioselective hydrolysis in which the (R) enantiomer of the amino acid amide remains and the S enantiomer is converted into the (S) amino acid, and the (S) amino acid is isolated.

2. Process according to Claim 1 in which the reaction mixture obtained after the conversion of the aminonitrile into the amino acid amide is subjected to treatment with a benzaldehyde, upon which the Schiff base of the amino acid amide is formed, the Schiff base is separated and is converted into the free amino acid amide.

3. Process according to Claim 1 or Claim 2, in which the (R)-amino acid amide is hydrolyzed to form the (R)-amino acid and the (R)-amino acid is recovered.

4. Process according to any one of Claims 1-3, in which R₁ and R₂ form a ring with 3 or 4 C atoms together with O atoms to which they are bound and the C atom to which the oxygen atoms are bound.

5. 2-Amino acid amide of formula 5 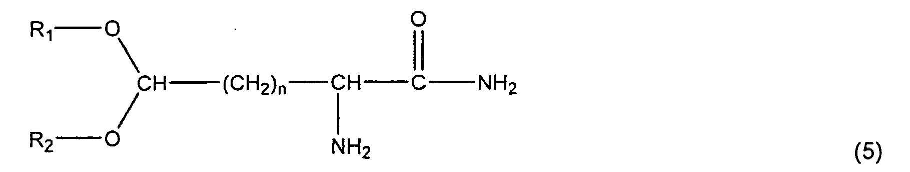 or formula 6, 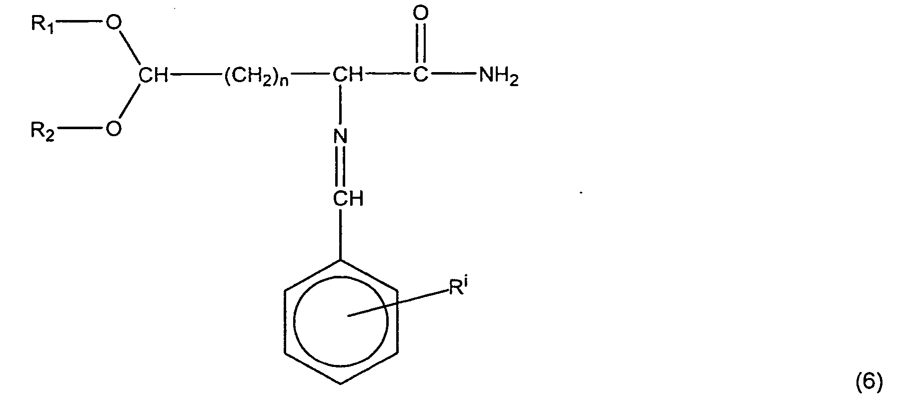 in which n equals 0, 1, 2, 3 or 4 and R₁ and R₂ each independently represent an alkyl group with 1-10 C atoms or form a ring with 3 or 4 C atoms together with the O atoms to which they are bound and the C atom to which the O atoms are bound, and Rⁱ represents 1-4 groups, each independently chosen from the group comprising halogen, nitro, an alkyl group or alkoxy group with 1-4 C atoms.

6. Optically active compound according to Claim 5 with an e.e. of > 95%.

7. Optically active compound according to claim 6 with an e.e. of > 98%.

8. Compounds according to any one of Claims 5-7, in which n equals 2 or 3.

9. Compounds according to any one of Claims 5-8, in which R₁ and R₂ form a ring with 3 or 4 C atoms together with the O atoms to which they are bound and the C atom to which the O atoms are bound.

## Patentansprüche

1. Verfahren zum Herstellen eines (S)-2-Amino-ω-oxoalkansäurederivats der Formel 1, 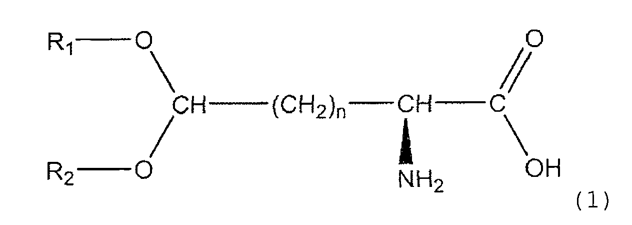 worin n gleich 0, 1, 2, 3 oder 4 und R₁ und R₂ jeweils unabhängig eine Alkylgruppe mit 1-10 C-Atomen darstellen oder einen Ring mit 3 oder 4 C-Atomen bilden, zusammen mit den O-Atomen, an welche sie gebunden sind, und dem C-Atom, an welches die O-Atome gebunden sind, **dadurch gekennzeichnet, dass** der entsprechende Aldehyd der Formel 2 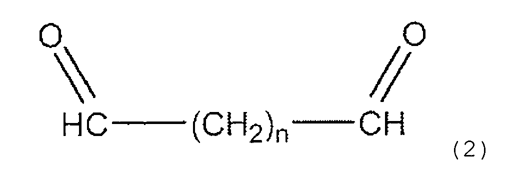 worin n wie oben beschrieben ist, umgewandelt wird in das entsprechende Acetal-geschützte Aldehyd der Formel 3 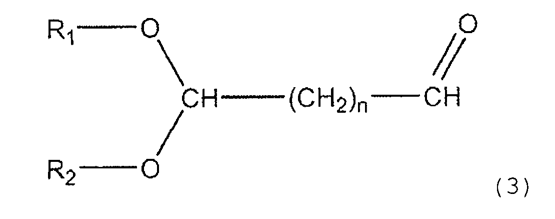 worin n, R₁ und R₂ wie oben beschrieben sind, der Acetal-geschützte Aldehyd umgewandelt wird in das entsprechende Aminonitril der Formel 4 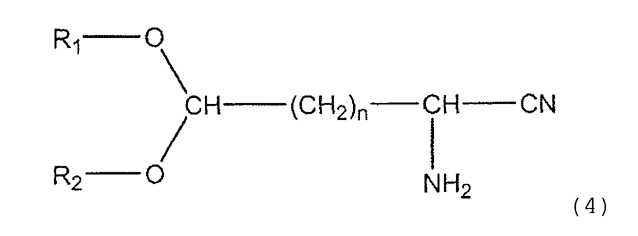 worin n, R₁ und R₂ wie oben beschrieben sind, das Aminonitril umgewandelt wird in das entsprechende Aminosäureamid der Formel 5 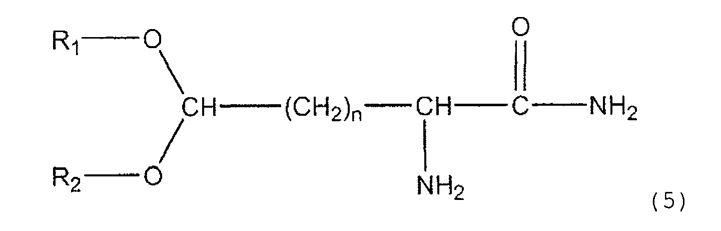 worin n, R₁ und R₂ wie oben beschrieben sind, das Aminosäureamid einer enzymatischen enantioselektiven Hydrolyse unterworfen wird, wobei das (R)-Enantiomer des Aminosäureamids verbleibt und das S-Enantiomer umgewandelt wird in die (S)-Aminosäure, und die (S)-Aminosäure isoliert wird.

2. Verfahren gemäß Anspruch 1, wobei das nach der Umwandlung des Aminonitrils in das Aminosäureamid erhaltene Umsetzungsgemisch einer Behandlung mit einem Benzaldehyd unterworfen wird, woraufhin die Schiff-Base des Aminosäureamids gebildet wird, die Schiff-Base abgetrennt wird und in das freie Aminosäureamid umgewandelt wird.

3. Verfahren gemäß Anspruch 1 oder Anspruch 2, wobei das (R)-Aminosäureamid hydrolysiert wird, um die (R)-Aminosäure zu bilden und die (R)-Aminosäure gewonnen wird.

4. Verfahren gemäß einem der Ansprüche 1-3, wobei R₁ und R₂ einen Ring mit 3 oder 4 C-Atomen bilden, zusammen mit den O-Atomen, an welche sie gebunden sind, und dem C-Atom, an welches die Sauerstoffatome gebunden sind.

5. 2-Aminosäureamid der Formel 5 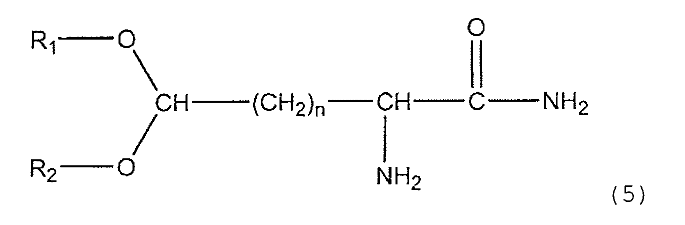 oder Formel 6 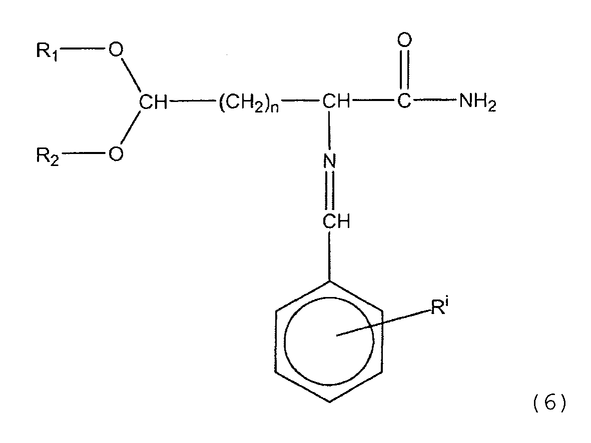 worin n gleich 0, 1, 2, 3 oder 4 und R₁ und R₂ jeweils unabhängig eine Alkylgruppe mit 1-10 C-Atomen darstellen oder einen Ring mit 3 oder 4 C-Atomen bilden, zusammen mit den O-Atomen, an welche sie gebunden sind, und dem C-Atom, an welches die O-Atome gebunden sind, und Rⁱ 1-4 Gruppen darstellt, jede unabhängig ausgewählt aus der Gruppe umfassend Halogen, Nitro, eine Alkylgruppe oder Alkoxygruppe mit 1-4 C-Atomen.

6. Optisch aktive Verbindung gemäß Anspruch 5 mit einem e.e. von >95%.

7. Optisch aktive Verbindung gemäß Anspruch 6 mit einem e.e. von >98%.

8. Verbindungen gemäß einem der Ansprüche 5-7, worin n gleich 2 oder 3.

9. Verbindungen gemäß einem der Ansprüche 5-8, worin R₁ und R₂ einen Ring mit 3 oder 4 C-Atomen bilden, zusammen mit den O-Atomen, an welche sie gebunden sind, und dem C-Atom, an welches die O-Atome gebunden sind.

## Revendications

1. Procédé de préparation d'un dérivé d'acide (S)-2-amino-ω-oxoalcanoïque de formule 1, 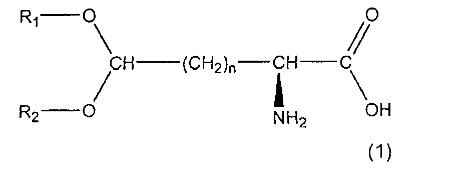 dans laquelle n vaut 0, 1, 2, 3 ou 4 et R₁ et R₂ représentent chacun indépendamment un groupe alkyle ayant 1 à 10 atomes de C ou forment un cycle avec 3 ou 4 atomes de C conjointement avec les atomes de O auxquels ils sont liés et l'atome de C auquel les atomes de O sont liés, **caractérisé en ce que** l'aldéhyde correspondant de formule 2 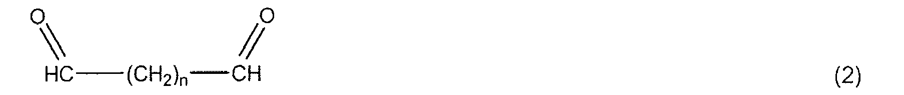 avec n tel que décrit ci-dessus est converti en l'aldéhyde correspondant protégé avec un acétal de formule 3 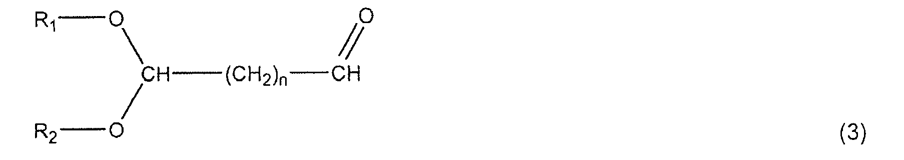 avec n, R₁ et R₂ tels que décrits ci-dessus, l'aldéhyde protégé avec un acétal est converti en l'aminonitrile correspondant de formule 4 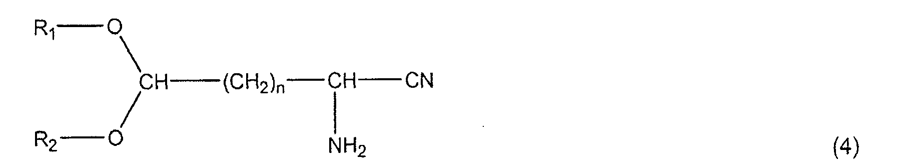 avec n, R₁ et R₂ tels que décrits ci-dessus, l'aminonitrile est converti en l'amide d'aminoacide correspondant de formule 5 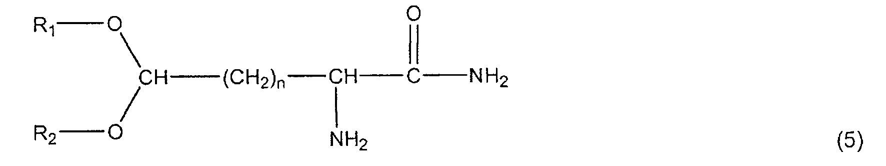 avec n, R₁ et R₂ tels que décrits ci-dessus, l'amide d'aminoacide est soumis à une hydrolyse enzymatique, énantiosélective dans laquelle l'énantiomère (R) de l'amide d'aminoacide reste et l'énantiomère S est converti en le (S) aminoacide, et le (S) aminoacide est isolé.

2. Procédé selon la revendication 1, dans lequel le mélange réactionnel obtenu après la conversion de l'aminonitrile en l'amide d'aminoacide est soumis à un traitement avec un benzaldéhyde, moyennant quoi la base de Schiff de l'amide d'aminoacide est formée, la base de Schiff est séparée et est convertie en l'amide d'aminoacide libre.

3. Procédé selon la revendication 1 ou la revendication 2, dans lequel le (R)-amide d'aminoacide est hydrolysé pour former le (R) aminoacide et le (R) aminoacide est récupéré.

4. Procédé selon l'une quelconque des revendications 1 à 3, dans lequel R₁ et R₂ forment un cycle ayant 3 ou 4 atomes de C conjointement avec les atomes de O auxquels ils sont liés et l'atome de C auquel les atomes d'oxygène sont liés.

5. Amide de 2-aminoacide de formule 5 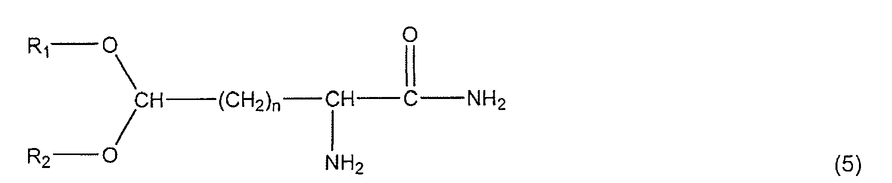 ou de formule 6 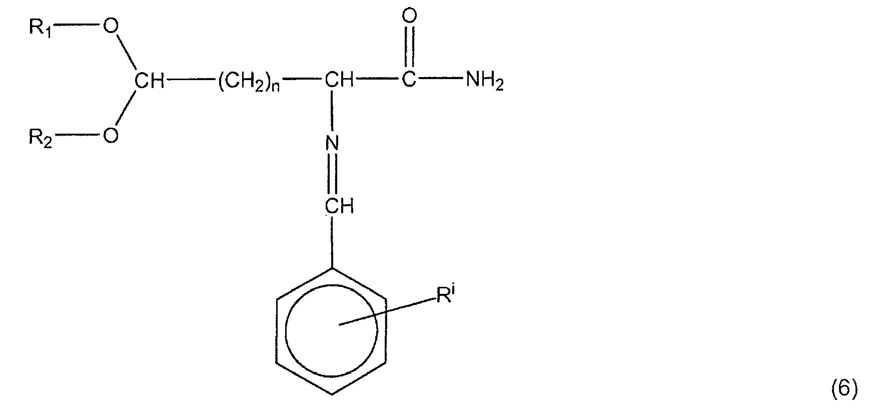 où n vaut 0, 1, 2, 3 ou 4 et R₁ et R₂ représentent chacun indépendamment un groupe alkyle ayant 1 à 10 atomes de C ou forment un cycle avec 3 ou 4 atomes de C conjointement avec les atomes de O auxquels ils sont liés et l'atome de C auquel les atomes de O sont liés, et R¹ représente 1 a 4 groupes, chacun choisi indépendamment dans le groupe comprenant un halogène, un groupe nitro, un groupe alkyle ou un groupe alcoxy ayant 1 à 4 atomes de C.

6. Composé optiquement actif selon la revendication 5 avec un e.e. qui est > 95 %.

7. Composé optiquement actif selon la revendication 6 avec un e.e. qui est > 98 %.

8. Composés selon l'une quelconque des revendications 5 à 7, dans lesquels n vaut 2 ou 3.

9. Composés selon l'une quelconque des revendications 5 à 8, dans lesquels R₁ et R₂ forment un cycle avec 3 ou 4 atomes de C conjointement avec les atomes de O auxquels ils sont liés et l'atome de C auquel les atomes de O sont liés.
